# EUROPEAN PATENT APPLICATION

(11) **EP 2 587 394 A1**
(43) Date of publication of application: **01.05.2013**
(21) Application number: 11187121.6
(22) Date of filing: 28.10.2011
(51) Int. Cl.: G06F 19/00

(54) **Mobile virtualization platform for the remote control of a medical device**

(71) Applicant: Debiotech S.A., 1004 Lausanne (CH)
(72) Inventor: Proennecke, Stephan, 1004 Lausanne (CH); François, Oscar, 1004 Lausanne (CH); Neftel, Frédéric, 1005 Lausanne (CH)
(74) Representative: Grosfillier, Philippe

(57) **Abstract**

Mobile device incorporating a virtualization platform comprising:
- a host operating system (hOS) emulating hardware components for at least one guest operating system (gOS),
- a first gOS handling common functions such as calendar or contacts, all those common functions being designed to be used in an uncontrolled environment,
- a medical operating system (mOs) handling remote control functions for a medical device, all those remote control functions being designed to be used in a controlled environment. There is a loopback process between mobile and medical device, where the medical device sends back the received configuration parameters to the mobile device to confirm the acceptance of these parameters.

## Description

### Field of invention

The present invention relates to the remote control of a medical device such as an insulin pump.

### State of the art

A remote control is required for controlling some medical devices like an insulin pump that is light and small like a patch pump, because it could be very difficult for the patient to see the content of a display that would be located on the pump itself. Most of the pumps today use a dedicated proprietary remote control, which represents another device to carry with all the disadvantages that it could generate like:
● To find a pocket to put it in a safe place with a fast and easy access.
● To not forget your remote control
● To think about charging it or to have spare batteries
● To prevent its deterioration due to a fall or any "bad" external condition, like exposure to the sun or to sand.

One way to prevent the use of another specific device is to integrate the remote control functionality into an existing device that the patient should already carry with him, such as blood glucose meter or a cell phone, which would have all the capabilities required for integrating the remote control features.

Using a cell phone for this purpose is very attracting but brings many security aspects that must be addressed before allowing its use for programming an insulin pump. Among the important security features that must be ensured are :
● Integrity of the data that are displayed to the user
● Integrity of the commands that are sent to the insulin pump
● Integrity and protection of the databases, which store the therapeutic parameters of the patient and the logs of the infusion history and the events.
● Responsiveness of the software at any time (eg: raising an alarm while another software has the focus, ability to process user requests while other tasks are overloading the resources like the CPU, etc).

### General description of the invention

The invention is set forth and characterized in the independent claims, while the dependent claims describe other characteristics of the invention.

The purpose of the invention is to offer a robust environment for ensuring the integrity of the data and the responsiveness of the medical device programming in the remote control.

To this effect the invention concerns a mobile device incorporating a virtualization platform comprising :
- a host operating system (hOS) emulating hardware components for at least one guest operating system (gOS),
- a first gOS handling common functions such as calendar or contacts, all those common functions being designed to be used in an uncontrolled environment,
- a medical operating system (mOs) handling remote control functions for a medical device, all those remote control functions being designed to be used in a controlled environment.

In the present text the expression "controlled environment" has to be understood as a space where:
● the responsiveness of the intended application is deterministic
● the list and version of the software packages and the operating system are known and can't be changed by the users
● the access to the hardware components is controlled and guaranteed
● the responsiveness of the hardware components (CPU, memory, RF link, etc) is deterministic
● a predefined minimum bandwidth is always garanteed to access hardware components (eg: CPU, network RF link, etc)

The controlled and uncontrolled environments are totally isolated.

As a consequence, the uncontrolled environment has no visibility on the interactions between the hardware and the controlled environment

In one embodiment the mobile device according to the invention is a cell phone. Any suitable OS can be used, for instance Android.

Advantageously the remote control functions are designed for the remote control of an insulin pump.

In another embodiment the mobile device is used in combination with a medical device. This assembly may advantageously comprise a loopback mechanism between both objects (e.g. insulin pump and remote control).

The present invention offers in particular the following advantages:
- Ensuring that the value programmed in the medical device (for instance an insulin pump) corresponds to the value expected by the user on the remote control. The user acknowledges the value preferably by entering a PIN code on the remote control.
- The invention also provides a controlled environment in which the responsiveness, the integrity and the security are assured by the core design of the low level operating system architecture.
- The proposed solution provides a secured environment, which may for instance prevent any unwanted application that could mimic the normal use by changing the therapy, like programming several additional infusions not wanted by the patient.

### Detailed description of the invention

The invention is discussed below in a more detailed way with examples illustrated by the following figures:
**Figure 1** shows the display of a mobile device according to the invention, which includes a virtualization platform.
**Figure 2** shows the overall architecture of a preferred embodiment of the invention, namely an assembly comprising a remote control (mobile device) and an insulin pump (medical device).
**Figure 3** illustrates a loopback mechanism according to the invention

The use of a mobile virtualization platform offers the possibility to divide the mobile device, e.g. a smartphone, into a controlled environment, e.g. for the remote control of the insulin pump, and an uncontrolled environment for general purpose tasks. The virtualization platform can be defined via a virtual machine application.

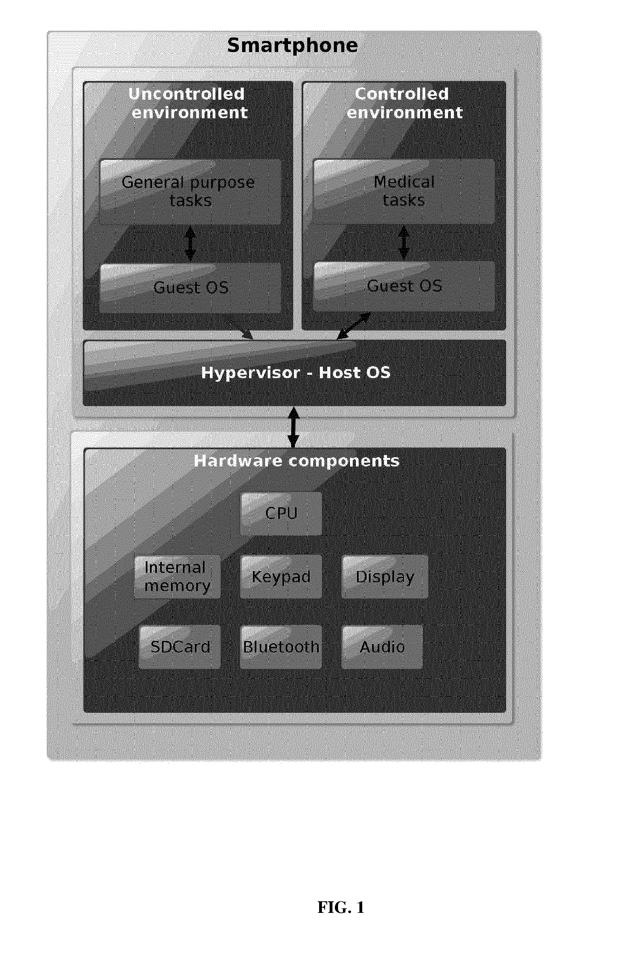

The architecture below describes a non-limitating example of a virtualization platform according to the invention (see figure 1) :
● a host Operating System (OS) emulating the hardware components to one or several guest OS (only 2 guest OS are illustrated on figure 1).
● one guest OS handling the general purpose tasks (eg: calendar, contacts, web browsing, phone communication, entertainment, etc) in a uncontrolled environment
● one guest OS handling the interaction with the insulin pump in a controlled environment

By using this architecture, the controlled environment has always the full control of the remote control in order to prevent any malicious application either to intercept or to modify or to generate commands / information exchanged with the pump. A typical action of such a malicious application would be to steal the PIN code of the user in order to mimic the programming of an infusion.

In addition to this architecture, a specific monitoring program can be implemented to check all running tasks in the controlled environment, which can disable any application that is not within a specific list of authorized application. This monitor may also able to measure the running time used by the application and indicate to the user any suspect overload of activity by triggering an alarm.

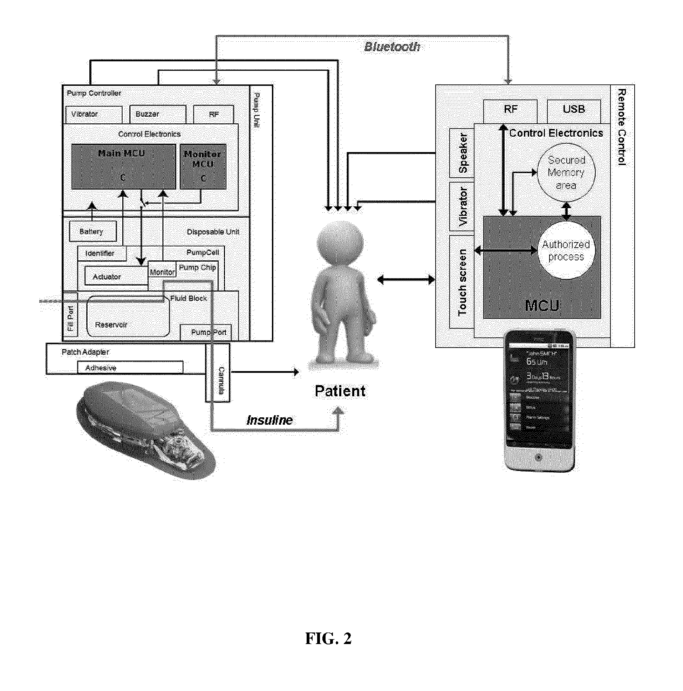

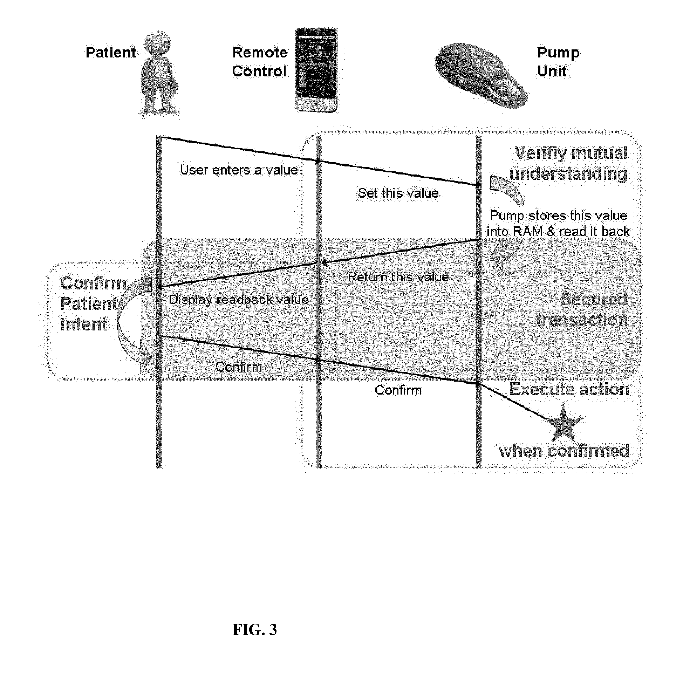

The next paragraphs relate to a preferred embodiment of the invention which comprises a loopback mechanism. This feature may provide a secure communication between the pump and the remote control, by taking into account that the architecture disclosed previously or a similar level of security is provided inside the remote control in order to ensure a secured bridge between the assembly according to the invention and the information read or entered by the patient. Figures 2 and 3 illustrate the use of a loopback mechanism with the mobile device according to the invention.

Preferably a direct secured pipe is created between the pump memory and a secured buffer on the remote control, which contains the displayed values. Then an authorized application on the remote control displays the value and records a user authentication, which will be used to construct the return value, which is sent back to the pump.

The secured pipe is open when the user has finished defining the parameters that he wants to program on the pump. It is closed when the user has acknowledged the parameters in order to allow the pump using them.

The next section will bring a more detailed description of the architecture and the secured loopback process.

The loopback process according to the present invention preferably requires the implementation of the following elements:
● A secured memory area in the pump
● A secured process in the pump that manages the encrypted communication of data between the secured memory area of the pump to the remote control.
● A secured display memory area in the remote control
● A secured process on the remote control that manages the encrypted communication of data between the pump to the secured display memory area of the remote control.
● A secured and authorized process on the remote control that transfers the data from the secured display memory area to the display of the remote control and builds the acknowledgement ticket of the user.

The architecture of these different elements is illustrated in Fig. 2.

The loopback process is initiated when the pump has received a set of parameters, which will change the set-up of the therapy or any security feature like the alarm settings.

The process preferably comprises the following steps:
**Done by the embedded software in the pump**
   - Write the parameters that must be acknowledged in the secured memory area of the pump
   - Generate a random information, commonly named a challenge
   - Open a secure pipe between the pump and the remote control
   - Indicate to the user that the pump is in loopback mode by means, for example, of a vibration on the pump itself
   - Send the parameters encrypted by using an encryption key Kp and the challenge to the remote control.
**Done by the embedded software in the remote control**
   - Receive and write the encrypted parameters and the challenge to the secured display memory area of the remote control.
**Done by the authorized application in the remote control**
   - Decrypt the parameters by using the key K_{RC}, which is the corresponding key to Kp. These keys can be symmetric or asymmetric. The authorized application is validated by having the correct corresponding key K_{RC}.
   - Display the decrypted parameters in a "Summary" page.
   - Enter the PIN code of the user. The PIN can be entered while using a random array display on the remote control device in order to prevent any application that would mimic user actions or intercept this information.

According to such random array display, the numbers (from 0 to 9) would be displayed in a random order which would be different every time a PIN code shall be entered by the patient.
- Build the acknowledgement ticket that will confirm the acceptance of these parameters by using the challenge, the key K_{RC} and the entered PIN code.
- Write the ticket in secured display memory area of the remote control.

**Done by the embedded software in the remote control**
   - Send this ticket back to the pump.
**Done by the embedded software in the pump**
   - Calculate the expected ticket
   - Receive and validate the acknowledgement ticket coming from the remote control.

This process is illustrated in the Fig. 3.

When the ticket is validated the loopback process is closed and the pump is allowed to use the updated parameters.

This basic process can be more elaborated or part of a more complex scheme in order to improve the security of the secured pipe.

The invention is of course not limited to the illustrated examples discussed previously.

## Claims

1. Mobile device incorporating a virtualization platform comprising:
- a host operating system (hOS) emulating hardware components for at least one guest operating system (gOS),
- a first gOS handling common functions such as calendar or contacts, all those common functions being designed to be used in an uncontrolled environment,
- a medical operating system (mOs) handling remote control functions for a medical device, all those remote control functions being designed to be used in a controlled environment.

2. Mobile device according to claim 1 wherein the functions related to said mOS are available in said hOS.

3. Mobile device according to claim 1 wherein said mOS constitutes a second gOS.

4. Mobile device according to one of the previous claims wherein said first gOS is also handling wireless communication such as web browsing or phone communication.

5. Assembly comprising a mobile device according to one of the previous claims and a medical device, said assembly including a loopback mechanism between the mobile device and the medical device.

6. Assembly according to claim 5 comprising :
- a secured memory area in medical device,
- secured processing means in the pump that manages the encrypted communication of data between said secured memory area and the mobile device,
- a secured display memory area in the remote control,
- secured processing means in the mobile device that manages the encrypted communication of data between the pump and said display memory area,
- secured and authorized processing means on the remote control that transfers the data from the secured display memory area to the display of the mobile device and builds the acknowledgement ticket of the user.

7. Assembly according to claim 5 or 6 comprising a direct secured pipe between the medical device memory and a secured buffer of the mobile device, said secured pipe being designed to be open when the user has finished defining the parameters he wants to program on the medical device and being designed to be closed when the user has acknowledged said parameters.

8. Assembly according to claim 5, comprising a signal designed to inform the user that a loopback process has been initiated.

9. Assembly according to claim 5, comprising a monitoring application that disables any unauthorized application or that indicates to the patient any suspect activity of an authorized application.

10. Use of a virtualization platform, as defined in anyone of the previous claims.

11. Use of a virtualization platform according to claim 10 in a cell phone.

12. Use of a virtualization platform according to claim 10 in a remote control for a medical device, which remote control is able to exchange data with any external system

13. Use of a virtualization platform according to claim 10,11 or 12 with a loopback mechanism.

14. Use of a virtualization platform according to claim 13 comprising the following steps :
Done by the embedded software in the pump
o Write the parameters that must be acknowledged in the secured memory area of the pump
o Generate a challenge
o Open a secure pipe between the pump and the mobile device
o Indicate to the user that the pump is in loopback mode by means of a vibration, sound or any other method that informs the patient.
o Send the parameters encrypted by using an encryption key K_{P} and the challenge to the mobile device.
Done by the embedded software in the remote control
o Receive and write the encrypted parameters and the challenge to the secured display memory area of the mobile device.
Done by the authorized application in the remote control
o Decrypt the parameters by using the key K_{RC}, which is the corresponding key to K_{P}. These keys can be symmetric or asymmetric. The authorized application is validated by having the correct corresponding key K_{RC}.
o Display the decrypted parameters in a "Summary" page.
o Enter the PIN code of the user.
o Build the acknowledgement ticket that will confirm the acceptance of these parameters by using the challenge, the key K_{RC} and the entered PIN code.
o Write the ticket in secured display memory area of the mobile device.
Done by the embedded software in the remote control
o Send this ticket back to the pump.
Done by the embedded software in the pump
o Calculate the expected ticket
o Receive and validate the acknowledgement ticket coming from the mobile device.
When the ticket is validated the loopback process is closed and the medical device is allowed to use the updated parameters.

15. Use according to any of the previous claims 10 to 14 wherein the user has to enter a PIN code to confirm the entrance in loopback mechanism, such PIN code being entered on a random displayed array.
